# EUROPEAN PATENT APPLICATION

(11) **EP 2 151 200 A1**
(43) Date of publication of application: **10.02.2010**
(21) Application number: 09009805.4
(22) Date of filing: 29.07.2009
(51) Int. Cl.: A61B 17/122, A61B 17/128

(54) **Successive clipping device**

(30) Priority: 06.08.2008 JP 2008203046
(71) Applicant: FUJIFILM Corporation, Minato-ku Tokyo (JP)
(72) Inventor: Ilda, Takayuki, Ashigara-kami-gun Kanagawa (JP); Cui, Shengfu, Saitama, 331-9624 (JP)
(74) Representative: Klunker . Schmitt-Nilson . Hirsch

(57) **Abstract**

A successive clipping device (10) comprises: a manipulating wire (20) inserted in the sheath (16) and having a forward end portion (40) connected to a rearmost clip (12E) to pull a clip series including a plurality of clips (12: 12A,12B,12C,12D and 12E); and a manipulating portion connected to a proximal end of the sheath to push out, for the foremost clip (12A) that has retreated in the sheath (16), the manipulating wire (20) with respect to the sheath (16) toward a forward end of the sheath by a first amount of movement necessary for causing the foremost clip to protrude from the forward end of the sheath, and push out, for the subsequent clip, the manipulating wire (20) with respect to the sheath (16) toward the forward end of the sheath by a second amount of movement smaller than the first amount of movement, and necessary for causing the subsequent clip to protrude from the forward end of the sheath (16).

## Description

### BACKGROUND OF THE INVENTION

The present invention relates to an endoscopic clipping device used for stopping bleeding, closing a puncture, etc. in a living body or the like and, in particular, to a successive clipping device which allows a plurality of clips to be used in succession.

An endoscopic clipping device causes a clip to protrude from the forward end of an endocope inserted into a living body to pinch a bleeding portion or a portion to be treated after the removal of the lesion tissue with the clip, thereby stopping the bleeding,or closing the puncture. In a conventionally used endoscopic clipping device, a single clip is detachably attached to the forward end of a manipulating wire, and each time clipping is performed, the entire sheath is pulled out the endoscope, and the sheath is loaded with the next clip before being inserted into the endoscope again for the next clipping. In this way, the conventional clipping device involves a rather bothersome operation.

In this connection, JP 2006-187391 A discloses an endoscopic clipping device allowing successive clipping.

Such an endoscopic clipping device includes a long sheath that accommodates a plurality of clips inside the forward end thereof. The endoscopic clipping device causes only a foremost clip among a plurality of clips to protrude from a forward end of the sheath, and causes the clip to perform a clipping manipulation (clipping) for stopping bleeding, closing a puncture, marking, etc. Then, the sheath is pulled to a rear end side by a predetermined length, whereby a next clip is placed in a state capable of performing clipping (is placed in a usable (standby) state) and the clipping can be performed successively.

The use of such an endoscopic clipping device enables clipping to be performed successively, which eliminates the necessity of performing a complicated work in which the entire sheath is pulled out the endoscope every time clipping is performed and a next clip is set and inserted again into the endoscope for the next clipping.

In an endoscopic clipping device disclosed in JP 2006-187391 A, in most cases, a clip is placed in a state capable of performing clipping by pushing a wire toward a forward end side by a predetermined length. However, such an endoscopic clipping device has the following problem: even if the wire is pushed to the forward end side by a predetermined length in the same way, a clip may not be placed in a usable state, which makes it impossible to perform clipping.

### SUMMARY OF THE INVENTION

It is an object of the present invention to solve the above-mentioned problems in the related art and to provide a successive clipping device that can place a clip in a state capable of performing clipping stably at all the times.

A successive clipping device according to the present invention comprises: a plurality of clips each loaded in a forward end portion of a sheath, while being engaged with another of the clips connected respectively in a front-back direction; a plurality of connection rings corresponding to the plurality of individual clips, fitted in the sheath so as to be capable of advancing and retreating therein, and each covering an engagement portion between the corresponding clips to retain the clips in a connection state; a manipulating wire inserted in and through the.sheath, and having a forward end portion thereof connected to the rearmost clip to pull a clip series including the plurality of clips; and a manipulating portion connected to a proximal end of the sheath to push out, for the foremost clip that has retreated in the sheath, the manipulating wire with respect to the sheath toward a forward end of the sheath by a first amount of movement necessary for causing the foremost clip to protrude from the forward end of the sheath so as to bring the foremost clip into a usable state, and push out, for the subsequent clip, the manipulating wire with respect to the sheath toward the forward end of the sheath by a second amount of movement smaller than the first amount of movement, and necessary for causing the subsequent clip to protrude from the forward end of the sheath so as to bring the subsequent clip into the usable state.

### BRIEF DESCRIPTION OF THE DRAWINGS

FIGS. 1A and 1B are a partial side cross-sectional view and a partial front cross-sectional view, respectively, illustrating a successive clipping device according to Embodiment 1 of the present invention.
FIG. 2 is a perspective view illustrating a configuration of a clip used in Embodiment 1.
FIGS. 3A through 3C are a front view, a front cross-sectional view, and a bottom view of a connection ring used in Embodiment 1, respectively.
FIGS. 4A and 4B are a front view and a plan view, respectively, illustrating a schematic configuration of a manipulating portion used in Embodiment 1.
FIG. 5 illustrates an internal structure of the manipulating portion.
FIGS. 6A through 6E are partial cross-sectional views, respectively, illustrating the state of the successive clipping device in Embodiment 1 during clipping in stages.
FIGS. 7A and 7B are partial cross-sectional views, respectively, illustrating the predetermined state of the successive clipping device in Embodiment 1 during clipping.
FIG. 8 illustrates an internal structure of the manipulating portion used in Embodiment 2.

### DETAILED DESCRIPTION OF THE PREFERRED EMBODIMENTS

In the following, preferred embodiments of the clipping device of the present invention are described with reference to the accompanying drawings.

### Embodiment 1

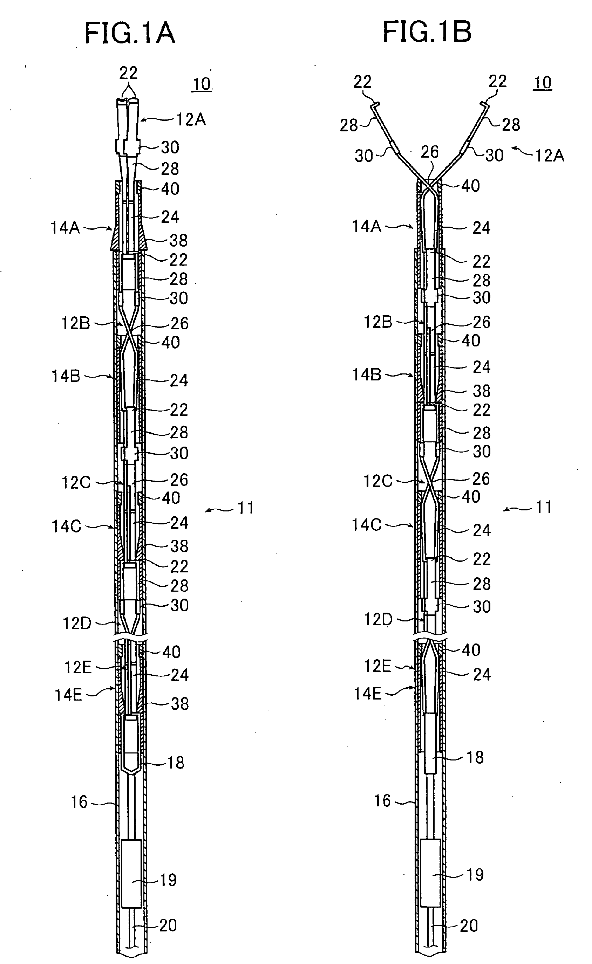

FIGS. 1A and 1B are sectional views of a clipping device 10 according to Embodiment 1 of the present invention, and FIG. 1B is a diagram as seen from an angle differing from FIG. 1A by 90 degrees. The clipping device 10 includes a manipulation operating portion 11. The manipulation operating portion 11 is composed of a plurality of clips 12 (12A, 12B, 12C, 12D, 12E), connection rings 14 (14A, 14B, 14C, 14D, 14E) that cover engagement portions of the adjacent clips 12 to maintain the connection state of the clips 12, a sheath 16 in which the clips 12 and the connection rings 14 are fitted, a dummy clip 18 connected to the trailing clip 12E, and a manipulating wire 20 connected to the dummy clip 18 via a connecting member 19.

FIGS. 1A and 1B illustrate an initial state (standby state) immediately before the start of clipping manipulation by the foremost clip 12.

One clip 12 and one connection ring 14 corresponding to the clip 12 form one endoscopic bleeding stop clip member, and the manipulation operating portion 11 includes a plurality of such bleeding stop clip members loaded into the interior of the distal end portion of the elongated sheath 16.

Further, the terminal end of the successive bleeding stop clip members is engaged with the dummy clip 18, and the dummy clip 18 is connected to the connecting member 19, through which the manipulating wire 20 and the plurality of clips 12 are connected to each other. Though not shown in FIGS. 1A and 1B, the manipulating wire 20 extends to the proximal end of the sheath 16 to be connected to a manipulating portion described later.

When the manipulating wire 20 is pulled out by a predetermined length by the manipulation of the manipulating portion, the series of clips 12 are moved by the same amount, and the foremost clip 12 is clamped by the connection ring 14 retaining the same, whereby clipping for stopping bleeding, marking, etc. is performed by the foremost clip 12. When the clipping by the foremost clip 12 has been completed, the sheath 16 is pulled to the manipulating portion side by a predetermined length, whereby the next clip 12 is placed in a usable state (standby state), thus making it possible to perform clipping successively.

While in FIGS. 1A and 1B the foremost clip 12A protrudes from the forward end of the sheath 16, when loading the plurality of clips 12 into the sheath 16, setting is made such that the foremost clip 12A is completely accommodated within the sheath 16. Further, while FIGS. 1A and 1B illustrate the 5-successive clipping device 10 in which 5 clips 12 are loaded in the sheath 16, the number of clips 12 may be any number not less than two.

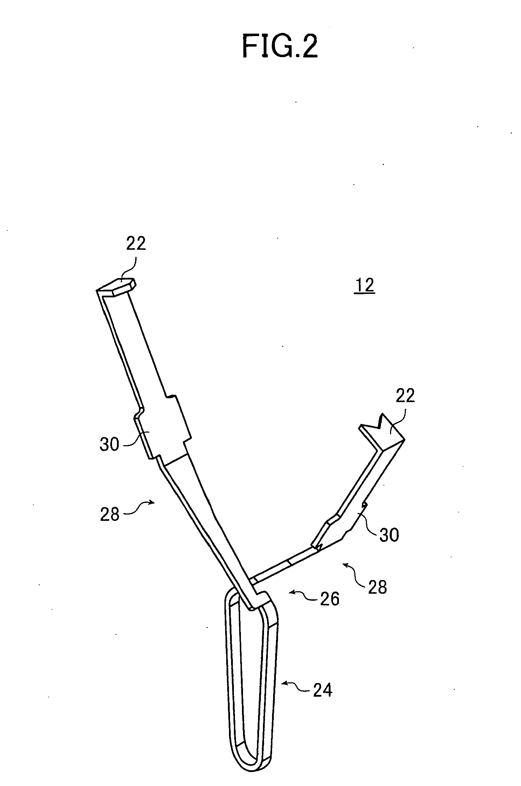

FIG. 2 is a perspective view of the clip 12. The clip 12 is a closed clip having a turned portion 24 turned by 180 degrees with respect to claw portions 22. That is, in forming the clip 12, a single elongated plate is bent by 180 degrees to form a closed end, and then both ends thereof are caused to cross each other at a crossing portion 26. Further, the end portions are bent so as to be opposed to each other, thereby forming the claw portions 22 to two open ends. On the open-end side of the crossing portion 26, there exist arm portions 28, 28, and, on the closed-end portion thereof, there exists the turned portion 24. At the central portion of each arm portion 28, 28, there are formed a partially widened.projections 30, 30. The clip 12 may be formed of a metal with biocompatibility. For example, it is possible to use SUS 631, which is a spring stainless steel.

In the clip 12, the forward end portion (a clamping portion 40 described later) of the connection ring 14 fitted onto the crossing portion 26 moves by a predetermined amount toward the claw portions 22, 22 while pressurizing the arm portions 28, 28, whereby the arm portions 28, 28 and the claw portions 22, 22 are closed, with the claw portions 22, 22 exerting a predetermined fit-engagement force.

To reliably pinch an object of a bleeding portion or a portion to be treated after the removal of the lesion tissue, the claw portions 22, 22 are formed as V-shaped male type and female type ones. Further, as illustrated in FIG. 2, the arm portions 28 of the clips 12 gradually increase in width from the crossing portion 26 toward the projections 30.

The projections 30 have a width larger than that of the portions of the distal end side openings and the proximal end side openings of the connection ring 14 abutted by the projections 30. Thus, while the portions of the clip 12 other than the projections 30 can enter the interior of the connection ring 14, the projections 30 cannot enter the interior either from the distal end side or the proximal end side of the connection ring 14.

As illustrated in FIGS. 1A and 1B, the claw portions 22 of the second clip 12B are engaged with the turned portion 24 of the first clip 12A and retained by the connection ring 14A in a closed state, whereby the first clip 12A and the second clip 12B are connected together. As illustrated in FIG. 1A, the claw portions 22, 22 of the second clip 12B are engaged with the turned portion 24 of the first clip 12A in a direction orthogonal thereto, with the first clip 12A and the second clip 12B being differing in orientation by 90 degrees. Similarly, the clips 12C, 12D, and 12E are connected together, with their orientations alternately differing by 90 degrees.

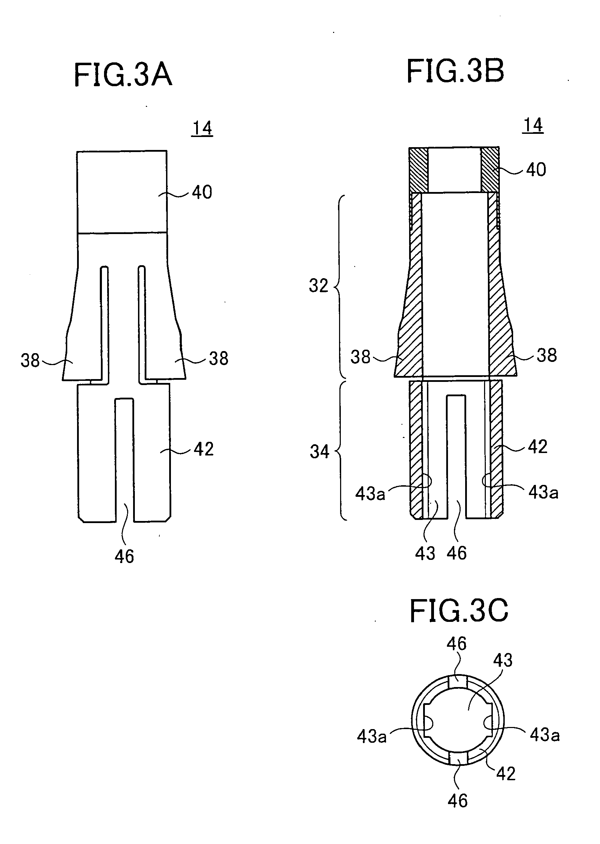

Each connection ring 14 is fitted into the sheath 16 so as to be capable of advancing and retreating while covering the engagement portion between two clips 12 and maintaining their connected state. That is, the outer diameter of the connection rings 14 is substantially the same as the inner diameter of the sheath 16 so that they can smoothly advance and retreat within the sheath 16 as the clips 12 move. FIGS. 3A through 3C illustrate the construction of each connection ring 14. FIG. 3A is a front view of the connection ring 14, FIG. 3B is a front cross-sectional view thereof, and FIG. 3C is a bottom view thereof.

The connection ring 14 includes a clamping portion 40 and a retaining portion 42. In the connection ring 14, the clamping portion 40 formed of metal is fixed to the forward end of the retaining portion 42 formed of resin, and the two members form an integral structure. The retaining portion 42 formed of resin serves to maintain the connected state and to retain the clip within the connection ring, and the clamping portion 40 formed of metal serves to clamp the clip. It is also possible for the connection ring 14 to be formed by a single member if it can provide the functions of both the clamping portion 40 and the retaining portion 42.

The clamping portion 40 is a cylindrical (ring-like) metal component mounted to the forward end side of the connection ring 14, and has a hole whose inner diameter is larger than the width of the portion of the clip 12 in the vicinity of the crossing portion 26 and smaller than the width of the projections 30. Thus, while the clamping portion 40 can move in the vicinity of the crossing portion 26 of the clip 12 it retains, it cannot be detached to the forward end side beyond the projections 30. That is, the projections 30 function as a stopper determining the movement limit of the connection ring 14 advancing with respect to the clip 12.

The clamping portion 40 is set at a predetermined position in the vicinity of the crossing portion 26 of the clip 12. The clamping portion 40 moves from its initial position, i.e., from the crossing portion 26 toward the projections 30, with the arm portions 28 of the clip 12 increasing in width, whereby it closes the arm portions 28, 28 of the diverging clip 12 to perform fixation and clamping. As the material of the clamping portion 40, a metal with biocompatibility, for example, a stainless steel SUS 304 is used. By forming the clamping portion 40 of metal, it is possible to exert on the metal clip 12 a frictional force, which serves as the clamping force.

The retaining portion 42 is a schematically cylindrical (ring-like) component formed by resin molding. The retaining portion 42 has a first region 32 retaining the preceding clip 12 and a second region 34 which is a connection retaining region retaining the next clip 12 while connected to the preceding clip.

The first region 32 has a large circular hole capable of accommodating the turned portion 24 of the clip 12 and larger than the hole of the clamping portion 40. On the outer surface of the forward end portion of the first region 32, there is formed a stepped portion onto which the clamping portion 40 is to be fitted, and the clamping portion 40 and the retaining portion 42 are fit-engaged with each other through close fit such that they are not detached from each other while loaded in the sheath 16 and during clipping manipulation. Further, the first region 32 has skirt portions 38 each diverging while inclined in a skirt-like fashion with respect to the axis of the connection ring 14 main body.

The forward end side, that is, the upper base portion of each skirt portion 38 as seen in FIGS. 3A and 3B is connected to the main body of the retaining portion 42, whereas the lower, diverging portion thereof is partially separated from the main body to be radially diverged or closed. Two skirt portions 38 are formed so as to be separated from each other by 180 degrees at the same position in the pulling direction for the clip 12, that is, in the vertical direction in FIGS. 3A and 3B.

When left as they are, that is, when in a state in which no external force is being imparted thereto, the skirt portions 38, 38 are diverged in a skirt-like fashion as illustrated in FIG. 3A. At this time, the interior of the first region 32 of the retaining portion 42 forms a columnar space as illustrated in FIG. 3B. When loading the connection rings 14 into the sheath 16, the following takes place: in the case, for example, of the second connection ring 14B illustrated in FIG. 1B, the skirt portions 38 are pushed in to enter the internal space, and the inner peripheral side portions of the skirt portions 38 pressurize the side surface (edge portion) of the turned portion 24 of the clip 12B retained by the first region 32, thus retaining the clip 12B such that it does not move in the rotating direction and the advancing/retreating direction within the connection ring 14B. It is also possible for the skirt portions 38 to pressurize and retain the clip retained by the second region 34, that is, the succeeding clip.

As in the case of the first connection ring 14A illustrated in FIG. 1A, the skirt portions 38, 38 extend beyond the forward end of the sheath 16, and are opened due to their own elasticity, releasing the retention of the clip 12A and becoming wider than the inner diameter of the sheath 16 to prevent the connection ring 14A from retreating into the sheath 16. In this state, the manipulating wire 20 is pulled, and the clip 12A retreats, whereby the connection ring 14A advances relative to the clip 12A to clamp the clip 12A.

Thus, it is necessary for the skirt portions 38 to have elasticity so that they can be closed inwardly within the sheath 16 and widen in a skirt-like fashion when they get out of the forward end of the sheath 16 and released from the external force. At the same time, it is also necessary for the skirt portions 38 to exhibit rigidity enabling them to retain the clip 12 within the sheath 16 and to withstand the repulsive force of the clamping force of the clip 12 at the forward end of the sheath 16.

From the above viewpoints, as the material of the retaining portion 42, there is used a material exhibiting biocompatibility and providing the requisite elasticity and rigidity for the skirt portions 38. As for their configuration, it is determined so as to satisfy the requirements in terms of elasticity and rigidity for the skirt portions 38. As the material of the retaining portion 42, it is possible to use, for example, polyphenylsulfone (PPSU). From the viewpoint of ease of production, it is desirable for the retaining portion 42 to be formed as an integral molding.

The second region 34 is provided on the proximal end side of the first region 32. The succeeding clip 12 engaged with the clip 12 retained by the first region 32 is retained in a state in which the claw portions 22, 22 thereof are closed while holding the closed end (tail portion) of the turned portion 24 of the preceding clip 12 therebetween.

The length of the second region 34 is substantially equal to the movement length required for the clamping portion 40 set at the initial position with respect to the clip 12 to move until the clamping of the clip 12 is completed. That is, while the clip 12 retreats relative to the connection ring 14 to be clamped, the second region 34 of the connection ring 14 maintains the connection between the two clips 12, 12 retained therein, allowing the pulling force of the rear clip 12 to be transmitted to the front clip 12, and when the clamping has been completed, the engagement portion of the two clips 12, 12 is detached from the second region 34, thereby canceling the connection between the clips 12, 12.

As illustrated in FIG. 3C, the second region 34 has a hole 43 having the same inner diameter as the proximal end side portion of the first region 32, and further, two grooves (recesses) 43a opposed to each other are formed. The grooves 43a, 43a can accommodate the arm portions 28, 28 of the clip 12 retained in the second region 34, with the claw portions 22, 22 being closed. Further, in the second region 34, slits 44 that are cut in from the proximal end are formed at two positions.

The grooves 43a, 43a are provided at two positions in the direction in which the claw portions 22 of the clip 12 retained in the second region 34 are opened and closed (horizontal direction in FIGS. 3B and 3C). The plate surfaces of the arm portions 28, 28 of the clip 12 retained in the second region 34 abut the inner walls of the grooves 43a, 43a. The width (opening width) of the grooves 43a is slightly larger than the maximum width of the arm portions 28 of the clip 12, and the distance from the wall surface of one groove 43a to the wall surface of the other groove 43a is substantially equal to the sum total of the lengths of the two claw portions 22, 22 of the clip 12 (length in the diverging direction). The width of the grooves 43a is smaller than the width of the projections 30 formed on the arm portions 28. Thus, the projections 30 of the clip 12 retained in the second region 34 cannot enter the grooves 43a.

The distance between the wall surfaces of the two grooves 43a is such that the engagement between the turned portion 24 of the preceding clip 12 and the claw portions 22, 22 of the next clip 12 is not canceled, and the distance is smaller than the sum total of the lengths of the two claw portions 22, 22 and the width of the portion of the turned portion 24 engaged with the claw portions 22, 22.

For example, the claw portions 22, 22 of the clip 12 retained in the second region 34 may slightly overlap each other, or the connection of the clip with the preceding clip 12 may be maintained, with a slight gap being left between the claw portions 22, 22.

The engagement portion between the two clips 12, 12 is retained in the portion of the second region 34 close to the boundary between the second region 34 and the first region 32. Inside the sheath 16, the turned portion 24 of the preceding clip 12 (e.g., the clip 12B in the connection ring 14B illustrated in FIG. 1B) is retained by the closed skirt portions 38 in the first region 32, and hence the advancing/retreating movement and rotating movement of the clip is restrained. The next clip 12 (e.g., the clip 12C in the connection ring 14B illustrated in FIG. 1B) engaged with the preceding clip 12 is retained in an orientation differing by 90 degrees from the preceding clip by the grooves 43a of the second region 34, whereby rotating movement of the clip is restrained, and the clip is engaged with the preceding clip restrained in advancing/retreating movement, thereby restraining the advancing/retreating movement thereof. That is, the engagement portion between the front and rear clips is retained by the connection ring 14 with very little play.

The slits 46 are formed at two positions deviated from the skirt portions 38, 38 by 90 degrees so as to be shallower than the upper end of the second region 34. In other words, the slits 46 are provided at positions deviated by 90 degrees from the direction in which the clips 12 retained by the second region 34 are diverged.

Due to the provision of the slits 46, the connection ring 14 is improved in terms of flexibility, and the clipping device 10 can pass a curved portion of small curvature. Further, due to the provision of the slits 46, the hem (proximal end portion) of the connection ring 14 is partially turned up, and hence, when the front and rear clips 12 are connected together prior to the loading of the clips 12 into the sheath 16, the connection is advantageously facilitated through the turning of the hem of the connection ring 14.

The slits 46 are situated so as to be shallower than the skirt portions 38, whereby a substantial reduction in the strength of the connection ring 14 is prevented. Further, the depth of the slits 46 is shallower than the position of the rear end of the clip 12 retained in the first region 32, that is, shallower than the engagement position of the clips 12, 12, and hence, also in the connection clip unit prior to the loading into the sheath 16, it is possible to maintain the retention of the clip 12 in the second region 34 of the connection ring 14.

As illustrated in FIGS. 1A and 1B, the claw portions 22 of the second clip 12B are engaged with the turned portion 24 of the first clip 12A, and the engagement portion is retained by the connection ring 14A. The claw portions 22, 22 of the second clip 12B are retained in the closed state by the inner wall of the connection ring 14A (second region 34 thereof). As a result, the connection of the first clip 12A and the second clip 12B is maintained. Similarly, the connection of the second clip 12B and the third clip 12C is maintained by the connection ring 14B, the connection of the third clip 12C and the fourth clip 12D is maintained by the connection ring 14C, the connection of the fourth clip 12D and the fifth clip 12E is maintained by the connection ring 14D, and the connection of the fifth clip 12E and the dummy clip 18 is maintained by the connection ring 14E.

The rearmost clip 12E is engaged with the dummy clip 18, which is not used for clipping. At its forward end portion, the dummy clip 18 has a resilient portion of a configuration similar to that of the open end side half as from the crossing portion of the clip 12. The resilient portion is engaged with the turned portion of the clip 12E, with the claw portions thereof being closed, and releases the clip 12E when the claw portions are opened. At the proximal end portion of the dummy clip 18, there exits the connecting member 19, to which the manipulating wire 20 is connected.

The sheath 16 is formed, for example, of a flexible coil sheath formed through intimate winding of metal wire. The sheath 16 allows the clip 12 to be movably fitted therein on the forward end side and accommodates the manipulating wire 20 connected to the clip 12 via the dummy clip 18 and the connecting member 19, and is connected to the manipulating portion on the proximal end side. The inner diameter of the sheath 16 is one allowing canceling of the engagement between the turned portion 24 of the preceding clip 12 and the claw portions 22, 22 of the next clip 12. That is, the inner diameter of the sheath 16 is larger than the sum total of the lengths of the two claw portions 22, 22 and the width of the portion of the turned portion 24 engaged with the claw portions 22, 22.

The manipulating wire 20 allows the plurality of clips 12 to advance or retreat in a series of clipping manipulations, and for example, is formed of a metal wire. The manipulating wire 20 is accommodated in the sheath 16. One end of the manipulating wire 20 is connected to the clip 12 via the connecting member 19 and the dummy clip 18, and the other end thereof extends to the proximal end of the sheath 16 to be connected to the manipulating portion. Further, the proximal end of the sheath 16 is also attached to the manipulating portion together with the manipulating wire 20.

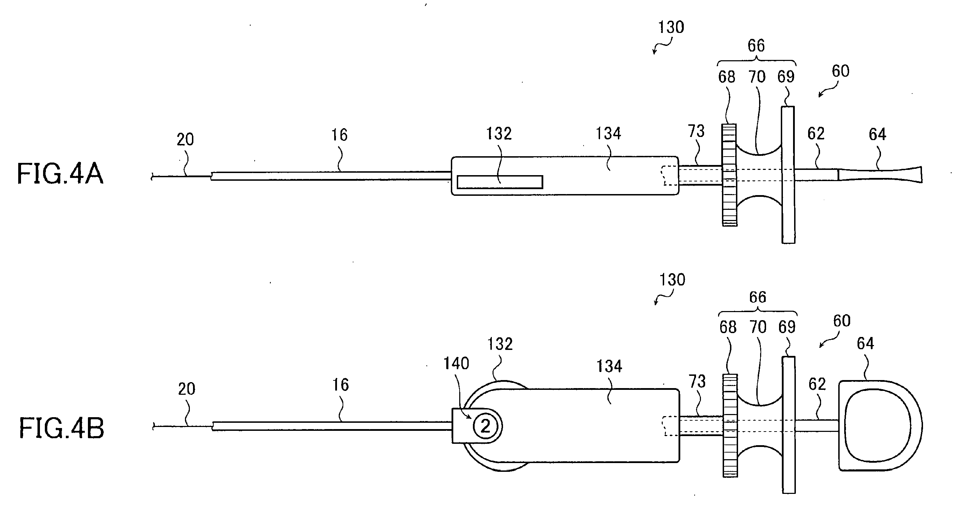

FIGS. 4A and 4B are a front view and a plan view, respectively, each illustrating a configuration of a manipulating portion 130 used in Embodiment 1. Of the manipulating portion 130, the left-hand part in the drawing is connected to the clips 12 and the sheath 16, the right-hand part in the drawing is manipulated by a manipulator. In the following description, the left-hand end portion and the right-hand end portion in each of FIGS. 4A and 4B are called a forward end and a proximal end, respectively.

The manipulating portion 130 has a case 134, and a wire operating portion 60 which pulls the manipulating wire 20 toward the proximal end by a predetermined amount.

The wire operating portion 60 includes a shaft-like member 62, and a manipulator element 66.

As indicated by the dotted lines in the drawings, one end portion of the shaft-like member 62 is fixed to the proximal end of the case 134, and a ring-shaped finger hook portion 64 through which the manipulator inserts his/her thumb during manipulation is formed at the other end portion thereof.

The manipulator element 66 is a spool-like member including a constricted portion 70 provided between two disk-shaped flanges 68 and 69 to be held between the forefinger and middle finger of the manipulator during manipulation.

Through the constricted portion 70 of the manipulator 66, the shaft-like member 62 has been slidably inserted and, to the forward end surface of the flange 68, a connecting element 73 for connecting a tray on which a wire roll described later is placed to the flange 68 is fixed.

By sliding the manipulator element 66 thus configured so as to advance and retreat with respect to the shaft-like member 62, it is possible to advance and retreat the connecting element 73, i.e., a wire send-out mechanism, and advance and retreat the manipulation wire 20, thereby allowing opening/closing manipulation of the clips 12 of the manipulation operating portion 11.

A mechanism for sliding the manipulator element 66 with respect to the shaft-like member 62 is not particularly limited. For example, by disposing a slider within the shaft-like member 62 so as to slide in the axial direction (extending direction) of the shaft-like member 62, and fixing a machine screw provided in the inner surface of the manipulator element 66 to the slider, it is possible to configure the mechanism such that the manipulator element 66 and the slider are integrally movable with respect to the shaft-like member 62 in the axial direction thereof.

During manipulation, the manipulator inserts his/her finger through the finger hook portion 64, holds the constricted portion 70 of the manipulator element 66 between his/her forefinger and middle finger, and pushes and pulls the manipulator element 66 with his/her forefinger and middle finger, whereby the manipulator element 66 is manipulated to be pushed and pulled with respect to the shaft-like member 62 in the axial direction thereof.

In this embodiment, the flange 68 of the manipulator element 66 is rotatable around the center of the shaft-like member 62. When the flange 68 is rotated, the connecting element 73, i.e., the wire send-out mechanism rotates to allow the rotation of the manipulating wire 20.

The case 134 has a display window 140. Through the display window 140, the numeral or symbol of a dial 132 described later can be viewed.

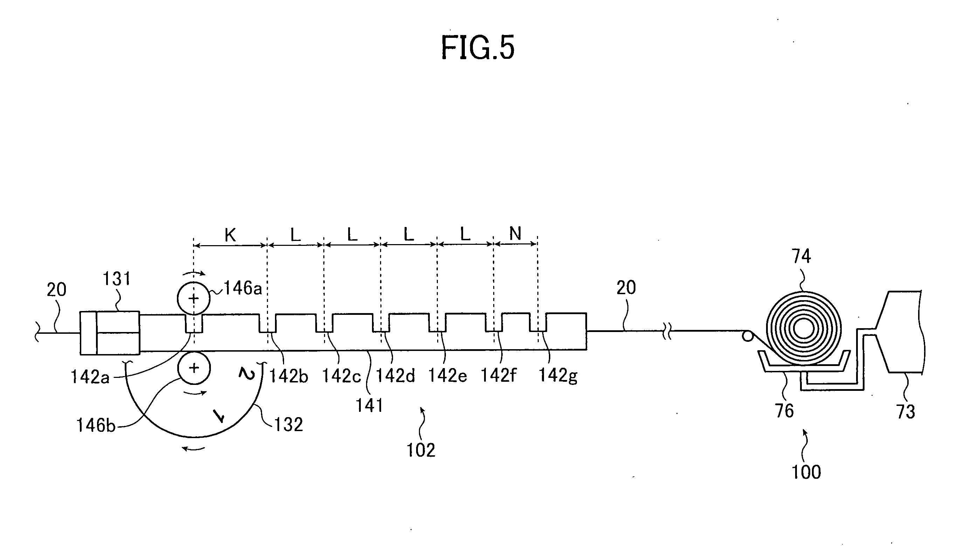

As illustrated in FIG. 5, the wire send-out mechanism 100 and a wire push-out mechanism 102 are disposed within the case 134.

The wire send-out mechanism 100 sends out the manipulating wire 20, and includes a wire roll 74, a tray 76, and the connecting element 73.

The wire roll 74 has a shaft (not shown), and is obtained by winding the manipulating wire 20 around the shaft.

The tray 76 supports the wire roll 74 placed thereon.

The connecting element 73 passes through the proximal end surface of the case 134 to connect the bottom surface of the tray 76 to the forward end surface of the flange 68 of the manipulator element 66 described above.

By pulling or pushing the manipulator element 66 described above in the extending direction (hereinafter referred to also as axial direction) of the case 134, the wire send-out mechanism 100 having a configuration as described above is similarly pulled or pushed. Further, the manipulating wire 20 is also pulled or pushed thereby in the same direction.

The wire push-out mechanism 102 pulls the manipulating wire 20 by a predetermined length at a time, and pushes the manipulating wire 20 toward the forward end of the manipulating portion 130 by a predetermined length at a time. The wire push-out mechanism 102 has a rotating grip portion 131, the dial 132 described above, a scale 141, a first roller 146a, and a second roller 146b.

The rotating grip 131 is configured to allow the manipulating wire 20 to pass therethrough, and rotate itself to rotate the manipulating wire 20, thereby allowing the orientation of the clip 12 which is opened at the forward end of the sheath 16 to be changed.

To the forward end of the scale 141, the manipulating wire 20 is connected through the intermediation of the rotating grip portion 131, while the manipulating wire 20 is fixed directly to the rear end thereof. The scale 141 is used to pull the manipulating wire 20 by a predetermined distance at a time, and push the manipulating portion 130 toward the forward end of the manipulating portion 130.

The scale 141 has first to seventh recesses 142a to 142g which are arrayed from the forward end toward the rear end such that the distance between the first and second recesses 142a and 142b has a predetermined length K, each of the respective intervals between the second and third recesses 142b and 142c, between the third and fourth recesses 142c and 142d, between the fourth and fifth recesses 142d and 142e, and between the fifth and sixth recesses 142e and 142f has a predetermined length L, and the distance between the sixth and seventh recesses 142f and 142g has a predetermined length N.

The predetermined lengths K, L, and N are described later in detail.

The first and second rollers 146a and 146b sandwich the scale 141 therebetween, and rotate in opposite directions to move the scale 141 by friction in a longitudinal direction.

The dial 132 has numerals, symbols, or the like engraved therein and arranged along the circumference thereof, and rotates in synchronization with the second roller 146b.

By displaying the numerical or symbol through the display window 140, the dial 132 can notify the manipulator which one of the successive clips 12 is currently used. Specifically, "0" represents that the clipping device 10 has been inserted in an endoscope, "1" to "5" represent the respective clips 12A to 12E, and "E" represents a state where the clip 12 is absent.

In the wire push-out mechanism 102 described above, when the dial 132 is rotated clockwise in FIG. 5, e.g., the second roller 146b rotates counterclockwise, and the first roller 146a rotates clockwise, each in synchronization therewith, whereby the scale 141 is conveyed in sandwiched relation toward the forward end to allow the manipulating wire 20 to be pushed toward the forward end.

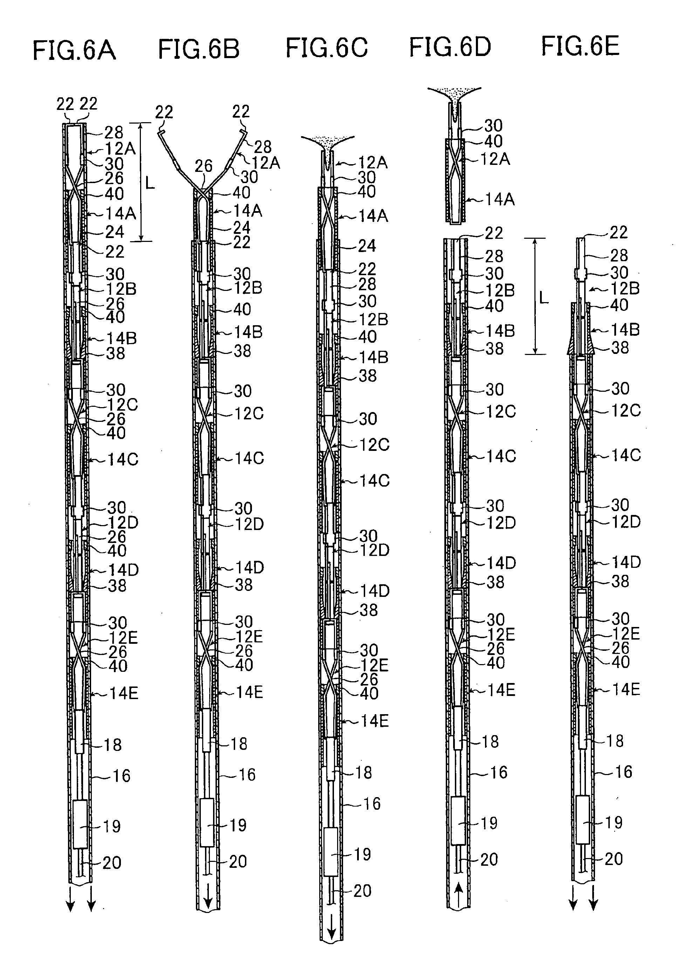

Next, the function of the clipping device 10 during an operation of clipping manipulation is described with reference to FIGS. 6A to 6E.

First, as illustrated in FIG. 6A, five breeding stop clip members (hereinafter referred to simply as clip members) including the clips 12A to 12E and the connection rings 14A to 14E are loaded in the sheath 16. Then, the sheath 16 is inserted into a forceps channel of the endoscope. At this time, as illustrated in FIG. 6A, the forward end of the clip 12A substantially corresponds to the forward end of the sheath 16.

The foremost (first-shot) clip 12A in the manipulation operating portion 11 is retained in a closed state by the inner wall of the sheath 16. The connection rings 14A to 14E are fitted such that the clamping portions 40 thereof are at respective initial positions in the vicinity of the crossing portions 26 of the clips 12A to 12E. At this time, the upper ends of the projections 30 of the clips 12B to 12E are positioned immediately below the respective connection rings 14A to 14D.

At the time when the clipping device is inserted in the endoscope, the first roller 146a of the manipulating portion 130 is engaged with the first recess 142a of the scale 141, as illustrated in FIG. 5. At this time, the numeral seen through the display window 140 is "0".

Then, the dial 132 is rotated clockwise so as to change the numeral seen through the display window 140 from "0" to "1", whereby the second roller 146b rotates counterclockwise, and the first roller 146a rotates clockwise, each in synchronization therewith, to convey the scale 141 in sandwiched relation toward the forward end till the first roller 146a is engaged with the second recess 142b. As a result, the scale 141 moves toward the forward end by the distance of the predetermined length K so that the manipulating wire 20 is also pushed out by the predetermined length K toward the forward end of the manipulating portion 130, and the foremost clip 12A protrudes from the sheath 16 to be in a usable state as illustrated in FIG. 6B.

Here, a description is given of the predetermined length K from the first recess 142a to the second recess 142b.

Conventionally, when the manipulating wire 20 is pushed out toward the forward end in order to bring the clip 12 loaded in the sheath 16 in a state as illustrated in FIG. 6A, i.e., the clip 12 having the forward end thereof substantially corresponding to the forward end of the sheath 16 into a manipulation possible state as illustrated in FIG. 6B, the manipulating wire 20 has been pushed out toward the forward end by the same length for each of the clips 12.

Specifically, as long as the scale 141 is used, the distances between the mutually adjacent recesses 142 are each set to have the length L of each of the clips 12 when loaded in the sheath 16 (hereinafter referred to simply as a load distance L), and the manipulating wire 20 is pushed out each time by the load distance L toward the forward end of the sheath 16.

In design, after the clipping device is inserted in the endoscope, the clip 12 is supposed to be surely brought into the usable state by pushing out the manipulating wire 20 toward the forward end by the load distance L. However, there has been a case where the clip 12 is not brought into the usable state, and clipping manipulation cannot be performed.

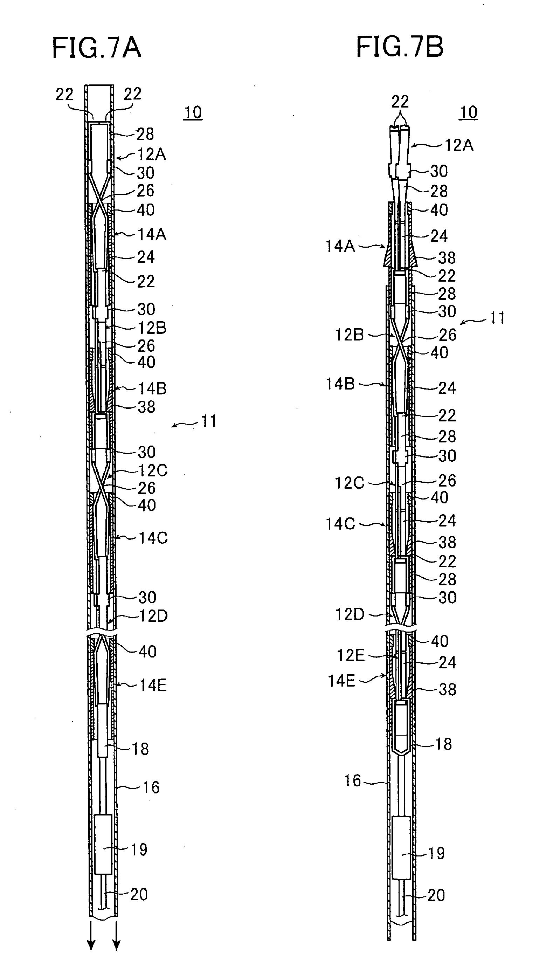

In view of this, the inventors of the present invention have vigorously studied the case, and made the following findings. That is, the forward end of the foremost clip 12A when the clipping device is inserted in the forceps channel of the endoscope is supposed to normally substantially correspond to the forward end of the sheath 16, as illustrated in FIG. 6A. However, because the forward end of the sheath is away from the proximal end thereof (side closer to the clipping manipulating portion) by several meters (e.g., about 2 meters), if the sheath 16 is curved midway, the manipulating wire 20 does not pass through the center portion of the sheath 16 in the curved section, but passes through the outside of the center portion in the sheath 16. As a result, the manipulating wire 20 does not reach a predetermined position in the sheath 16, and the foremost clip 12A may retreat into the sheath 16, as illustrated in FIG. 7A. In that case, even when the manipulating wire 20 is pushed out toward the forward end by the load distance L of the clip 12, the skirt portions 38 of the connection ring 14A corresponding to the clip 12A do not protrude completely from the sheath 16, and the clip 12A is not brought into the usable state.

Therefore, even in the case where the foremost clip 12A has retreated in the sheath 16, in order to allow the foremost clip 12A accommodated in the sheath 16 to be surely brought into a state where an operation of clipping manipulation is possible, the predetermined length K from the first recess 142a to the second recess 142b has been set to a value obtained by adding a pre-calculated amount of retreat of the clip 12A to the load distance L of the clip 12 on the preliminary assumption that the clip 12A has retreated in the sheath 16.

The distance between the forward end of the clip 12A that has retreated in the sheath 16 and the forward end of the sheath 16 (hereinafter referred to simply as an amount of retreat) differs depending on the curved state of the sheath 16, and is about 3.5 mm at a maximum.

For example, when the load distance of the clip 12 is 12.4 mm, and the pre-calculated amount of retreat of the clip 12A is 3.1 mm, the predetermined length K is 15.5 mm, which is the sum of 12.4 mm and 3.1 mm as the amount of retreat.

By thus setting the predetermined length K of the distance from the first recess 142a to the second recess 142b in the scale 141 to the value obtained by adding up the pre-calculated amount of retreat of the clip 12A and the load distance L of the clip 12, and rotating the dial 132 till the first roller 146a that has been engaged with the first recess 142a is engaged with the second recess 142b, the manipulating wire 20 can be moved toward the forward end by the predetermined length K. Even when the foremost clip 12A has retreated in the sheath 16, it is possible to surely bring the clip 12A into a clipping-operation possible state.

If the pre-calculated amount of retreat of the foremost clip 12A is smaller than an actual amount of retreat of the clip 12A for such a reason that the sheath 16 has not been so curved as expected, a gap is formed between the lower ends of the skirt portions 38 of the connection ring 14A corresponding to the clip 12A and the forward end of the sheath 16, as illustrated in FIG. 7B, when the engagement position of the first roller 146a is moved from the first recess 142a to the second recess 142b to push out the manipulating wire 20 toward the forward end of the manipulating portion 130.

In such a case, it is preferable to pull the manipulating wire 20 toward the proximal end, and eliminate the gap between the lower ends of the skirt portions 38 and the forward end of the sheath 16.

For example, in this embodiment, the gap between the lower ends of the skirt portions 38 and the forward end of the sheath 16 may be eliminated appropriately by pulling the manipulator element 66 of the wire operating portion 60 toward the proximal end, and thereby pulling the wire roll 74, i.e., the manipulating wire 20 toward the proximal end to pull the clip 12 back to the proximal end. At this time, the manipulating wire 20 wound in the wire roll 74 is held securely by a nip roller (not shown) or the like such that the length of the manipulating wire 20 does not change as a result of the sending out of the manipulating wire 20 from the wire roller 74. Further, the first roller 146a is positioned in the first recess 142a even after the adjustment.

On the other hand, also in the case of setting the distance between the first and second recesses 142a and 142b to the above-mentioned predetermined length K, if the skirt portions 38 of the foremost clip 12A do not open due to the twist of the manipulating wire 20 or the like, it is preferable to rotate the clip 12.

For example, in this embodiment, the manipulating wire 20 may be rotated appropriately by rotating the flange 68 either leftward or rightward to rotate the connecting element 73, i.e., the wire roll 74 or the rotating grip portion 131.

When the manipulating wire 20 is pushed out, a frictional force normally acts between the sheath 16 and the connection rings 14A to 14E fitted in the sheath 16. However, between the connection rings 14A to 14E and the clips 12A to 12E, a pressing force is exerted on the clips 12 by the inner portions of the closed skirt portions 38, and a pressing force resulting from a resilient force of the claw portions 22 of the rear-side clip 12 which are about to open is exerted on the inner wall surface of the second region 34 of the connection ring 14. Further, the projections 30 of the clips 12B to 12E abut on the proximal ends of the connection rings 14A to 14D, and cannot enter the holes 43 (see FIGS. 3B and 3C) of the connection rings 14. As a result, even when the manipulating wire 20 is pushed out, the connection ring 14A to 14E do not needlessly move. Therefore, the connection rings 14A to 14E can maintain their states of retaining the respective clips 12A to 12E.

When the skirt portions 38 of the foremost connection ring 14A have thus protruded from the forward end of the sheath 16, the skirt portions 38 of the connection ring 14A outwardly open, and the claw portions 22 of the clip 12A protruding from the sheath 16 are opened by a biasing force to be in the state of FIG. 6B. As a result, the first-shot clip 12A becomes usable. Because the connection portion between the clip 12A and the clip 12B is positioned immediately under the skirt portions 38 of the connection ring 14A, the forward end of the clip 12B substantially corresponds to the forward end of the sheath 16.

Next, the clipping device in the state of FIG. 6B is moved to press the diverged claw portions 22 of the clip 12A against a site desired to be subjected to clipping manipulation, and the manipulator element 66 of the manipulating portion 130 (see FIGS. 4A and 4B) is pulled, whereby the manipulating wire 20 is pulled toward the proximal end by a predetermined amount. By pulling the manipulating wire 20 toward the proximal end, all the clips 12A to 12E engaged in succession from the dummy clip 18 are uniformly pulled toward the proximal end. At this time, the manipulating wire 20 wound in the wire roll 74 is securely held by the nip roller (not shown) such that the length of the manipulating wire 20 does not change as a result of the sending out of the manipulating wire 20 from the wire roller 74.

At this time, in the states illustrated in FIGS. 6B and 6C, the skirt portions 38 of the connection ring 14A protruding from the forward end of the sheath 16 are opened, and the retention of the clip 12A by the pressure of the skirt portions 38 is cancelled. Further, because the skirt portions 38 are opened at the forward end of the sheath 16, the connection ring 14A is prevented from retreating into the sheath 16. Therefore, as illustrated in FIG. 6C, the foremost clip 12A retreats with respect to the connection ring 14A, when the manipulating wire 20 is pulled. The lower ends of the projections 30 of the clip 12A are pushed in the forward end of the connection ring 14A, i.e., the clamping portion 40, whereby clamping of the clip 12A by the connection ring 14A is completed.

The engaged portion between the clip 12A and the next clip 12B comes off from the rear end of the connection ring 14A at the same time as the clamping of the clip 12A by the connection ring 14A. When the engaged portion between the clip 12A and the clip 12B comes off from the connection ring 14A, the arm portions 28 of the clip 12B are diverged until they abut the inner wall of the sheath 16 due to a resilient force, and the claw portions 22, 22 of the clip 12B are diverged more widely than the width of the turned portion 24 of the clip 12A, whereby the connection between the clip 12A and the clip 12B is cancelled. This enables the clip 12A and the connection ring 14A to be detached from the sheath 16, whereby the clipping by the clip 12A and the connection ring 14A is completed.

On the other hand, the succeeding clips 12B through 12E are retained by the connection rings 14B through 14E whose skirt portions 38 are closed so as not to move in the rotating direction and the advancing/retreating direction with respect to the connection rings 14B through 14E. Further, the claw portions 22 are pressed against the inner walls of the second regions 34 (see FIG. 3B) of the connection rings 14B through 14E by the expanding force (urging force) of the claw portions 22 of the clips 12C through 12E engaged with the clips 12B through 12E and the claw portions of the dummy clip 18, with the result that the frictional force between the clips 12B through 12E and the connection rings 14B through 14E is enhanced. Thus, the connection rings 14B through 14E move with the movement of the clips 12B trough 12E.

That is, the clips and the connection rings other than the foremost clip 12A and the connection ring 14A retaining the same, i.e., the clips 12B through 12E and the connection rings 14B through 14E advance and retreat integrally with respect to the sheath 16, and the connected state of the clips 14B through 14E and the dummy clip 18 is maintained by the connection rings 14B through 14E.

The manipulating wire 20 is constructed so as to be capable of being pulled by a fixed amount from the initial state. This fixed amount is an amount equal to the length of the second regions 34 of the connection rings 14 or an amount slightly larger than that, and at the same time, it is an amount equal to the length from the lower ends of the projections 30 of each clip 12 to the forward end of the connection ring 14 retaining that clip 12, or an amount slightly smaller than that. In the manipulating portion 130 of FIG. 4A, this fixed amount is determined by the length as measured from the home position of the manipulator element 66 to the movement limit at the rear.

As described above, the manipulating portion 130 is configured such that the manipulator element 66 can be pulled by the given amount toward the proximal end, and the manipulating wire 20 can be pushed out by the same amount toward the forward end. Therefore, by pulling the manipulator element 66 together with the manipulating wire 20, the clip 12A is shifted from the state of FIG. 6B to the state of FIG. 6C. Then, by pushing the manipulator element 66 toward the forward end, the manipulating wire 20 is pushed out toward the forward end to shift the clip 12A from the state of FIG. 6C to the state of FIG. 6D. That is, the forward end of the second-shot clip 12B returns to the same position as illustrated in FIG. 6A which substantially corresponds to the forward end of the sheath 16.

At this time, the first roller 146a of the manipulating portion 130 is engaged with the second recess 142b.

Thus, after the first roller 146a is engaged with the second recess 142b, and the clip 12A has become usable, the manipulator element 66 of the manipulating portion 130 is pulled toward the proximal end by the given amount, whereby clamping with the clip 12A is completed. Next, when the manipulator element 66 is pushed toward the forward end, and clipping manipulation is thereby completed, the first roller 146a is at the position of the recess 142b with which the first roller 146a has been engaged when the clip 12A has become usable.

Next, in the manipulating portion 130, the dial 132 is rotated clockwise so as to shift the numeral seen through the display window 140 from "1" to "2" and, in synchronization therewith, the second roller 146b is rotated counterclockwise, and the first roller 146a is rotated clockwise, whereby the scale 141 is conveyed until the engagement position of the first roller 146a shifts from the second recess 142b to the third recess 142c. Consequently, the manipulating wire 20 is pushed out by the wire push-out mechanism 102 toward the forward end of the manipulating portion 130 by the distance between the second and third recesses 142b and 142c, i.e., by the predetermined length L, and brings the clip 12B into the usable state, as illustrated in FIG. 6E.

As illustrated in FIG. 6D, the predetermined length L is the distance between the respective forward ends of the preceding and subsequent two clips 12 loaded in the sheath 16, i.e., the load distance of the clip 12 in the sheath 16.

The retreat of the foremost clip 12A into the sheath 16 has an effect only in the case of bringing the foremost clip 12A into the usable state. Therefore, by setting the distance from the first recess 142a to the second recess 142b in the scale 141 to the predetermined length K, and offsetting the amount of retreat of the foremost clip 12A into the sheath 16 as described above, the second-shot and subsequent clips 12 are prevented from retreating into the sheath 16 when manipulation with the clip 12A is completed. Therefore, the manipulating wire 20 may be pushed out appropriately from the sheath 16 by the load interval of the clip 12 in the sheath 16.

Thereafter, in the same manner as with the clip 12A, the claw portions of the clip 12B are pressed against a site desired to be subjected to clipping manipulation, and the manipulator element 66 of the manipulating portion 130 is pulled toward the proximal end by the given amount, whereby clamping of the clip 12B by the connection ring 14B is completed. Then, by pushing out the manipulator element 66 together with the manipulating wire 20 toward the forward end, and disconnecting the clips 12B and 12C, clipping manipulation with the clip 12B is completed.

At this time, the first roller 146a of the manipulating portion 130 is at the position of the third recess 142c.

With the clips 12C, 12D, and 12E also, clipping manipulation is performed in the same manner as with the clip 12B.

After clipping manipulation with the rearmost clip 12E is completed, i.e., after the use of all the clips 12 has ended, the dial 132 is rotated clockwise so as to change the numeral seen from the display window 140 from "5" to "E" and, in synchronization therewith, the second roller 146b is rotated counterclockwise, and the first roller 146a is rotated clockwise to convey the scale 141 toward the forward end until the engagement position of the first roller 146a moves from the sixth recess 142f to the seventh recess 142g. That is, by causing the rollers 146a and 146b to move the scale 141 toward the forward end by the distance of the predetermined length N, the manipulating wire 20 is pushed out toward the forward end of the manipulating portion 130 by the predetermined length N. In this manner, the dummy clip 18 is caused to protrude from the forward end of the sheath 16, and disengaged from the manipulating wire 20.

The predetermined length N corresponds to a distance over which the manipulating wire 20 is moved toward the forward end such that, after clipping manipulation with the rearmost clip 12E has ended, the dummy clip 18 protrudes from the forward end of the sheath 16, and comes to a retrievable position.

For example, in a state where the use of all the clips 12 has ended, the forward end of the dummy clip 18 normally substantially corresponds to the forward end of the sheath 16. Therefore, it is appropriate to determine the travel distance of the manipulating wire 20, which is necessary for moving the dummy clip 18 with the forward end thereof corresponding to that of the sheath 16 to the retrievable position, and use the determined distance as the predetermined length N.

### Embodiment 2

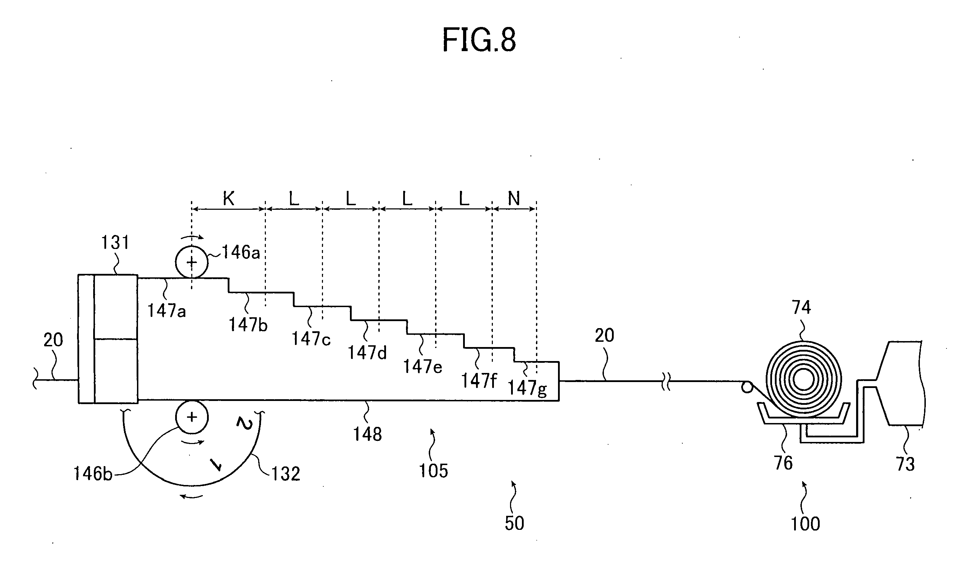

FIG. 8 illustrates an inner configuration of a manipulating portion 50 used in a successive clipping device according to Embodiment 2.

In order to avoid intricacy of description, Embodiment 2 is described by laying emphasis on the configuration and function of only a portion thereof different from Embodiment 1 described above.

The manipulating portion 50 in Embodiment 2 includes a wire send-out mechanism 100, and a wire push-out mechanism 105.

The wire send-out mechanism 100 has the same configuration as that of the wire send-out mechanism used in the manipulating portion 130 in Embodiment 1 illustrated in FIG. 5.

The wire push-out mechanism 105 includes the rotating grip portion 131, a scale 148, the dial 132, the first roller 146a, and the second roller 146b.

The rotating grip portion 131, the dial 132, and the first and second rollers 146a and 146b have the same configurations as those of the corresponding members in Embodiment 1 illustrated in FIG. 5.

The forward end of the scale 148 is connected to the manipulating wire 20 through the intermediation of the rotating grip portion 131, while the proximal end thereof is fixed directly to the manipulating wire 20. The scale 148 is used to pull the manipulating wire 20 by a predetermined length at a time, and push it out toward the forward end of the manipulating portion 50.

The scale 148 has a stepped upper surface. In the drawing, the distance from the center of a first plane 147a at an uppermost position to the center of a second plane 147b at a second uppermost position is set to have the predetermined length K, the respective distances from the center of the second plane 147b to the center of a third plane 147c at a third uppermost position, from the center of the third plane 147c to the center of a fourth plane 147d at a fourth uppermost position, from the center of the fourth plane 147d to the center of a fifth plane 147e at a fifth uppermost position, and from the center of the fifth plane 147e to the center of a sixth plane 147f at a sixth uppermost position are each set to have the predetermined length L, and the distance from the center of the sixth plane 147f at the sixth uppermost position to the center of a seventh plane 147g at a seventh uppermost position is set to have the predetermined length N.

Each of the predetermined lengths K, L, and N is the same as in Embodiment 1.

In the wire push-out mechanism 105, when the dial 132 is rotated clockwise, e.g., the second roller 146b rotates counterclockwise, and the first roller 146a rotates clockwise, each in synchronization therewith, to convey the scale 148 in sandwiched relation toward the forward end, whereby the manipulating wire 20 is pushed out toward the forward end.

The function of Embodiment 2 is different from that of Embodiment 1 only in the function of the wire push-out mechanism 105.

At the time when the clipping device is inserted into the endoscope, the first roller 146a is positioned at the center of the first plane 147a of the scale 148, as illustrated in FIG. 8.

At this time, the numeral of the dial 132 seen through the display window 140 is "0".

Then, the dial 132 is rotated clockwise so as to change the numeral of the dial 132 seen through the display window 140 from "0" to "1" and, in synchronization therewith, the second roller 146b is rotated counterclockwise, and the first roller 146a is rotated clockwise, whereby the scale 148 is conveyed in sandwiched relation such that the position of the first roller 146a moves from the center of the first plane 147a to the center of the second plane 147b of the scale 148. As a result, the scale 148 moves toward the forward end by the predetermined length K so that the manipulating wire 20 is pushed out toward the forward end by the predetermined length K, and brings the foremost clip 12A into a usable state as illustrated in FIG. 6B.

The clip 12A that has become capable of manipulation performs manipulation in the same manner as in Embodiment 1, and completes the manipulation. After the clip 12A has become usable, the manipulator element of the manipulating portion 50 is pulled toward the proximal end by a given amount, whereby clamping with the clip 12A is completed, as illustrated in FIG. 6C. Then, by pushing the manipulator element toward the forward end, clipping manipulation is completed, as illustrated in FIG. 6D.

Then, in the manipulating portion 50, the dial 132 is rotated clockwise so as to shift the numeral seen through the display window 140 from "1" to "2" and, in synchronization therewith, the second roller 146b is rotated counterclockwise, and the first roller 146a is rotated clockwise, whereby the scale 148 is conveyed in sandwiched relation toward the forward end such that the position of the first roller 146a moves from the center of the second plane 147b to the center of the third plane 147c of the scale 148. As a result, the manipulating wire 20 is pushed out toward the forward end by the predetermined length L, and brings the clip 12B into the usable state, as illustrated in FIG. 6E.

With respect to the clip 12B that has become capable of manipulation, clipping manipulation is performed in the same manner with the clip 12A.

With the clips 12C, 12D, and 12E also, clipping manipulation is performed in the same manner as with the clip 12B.

After clipping manipulation with the rearmost clip 12E is completed, i.e., after the use of all the clips 12 has ended, the dial 132 is rotated clockwise so as to change the numeral seen through the display window 140 from "5" to "E" and, in synchronization therewith, the second roller 146b is rotated counterclockwise, and the first roller 146a is rotated clockwise. As a result, the scale 148 is conveyed in sandwiched relation toward the forward end such that the position of the first roller 146a moves from the sixth plane 147f of the scale 148 toward the seventh plane 147g thereof, and the manipulating wire 20 is pushed out toward the forward end by the predetermined length N. In this manner, the dummy clip 18 is caused to protrude from the forward end of the sheath 16, and detached from the manipulating wire 20.

Thus, as described above, in the present invention, in order to bring the foremost clip 12A into the usable state, the distance over which the manipulating wire 20 is pushed out toward the forward end is set to have the predetermined length K, i.e., a length obtained by adding up the amount of retreat of the foremost clip 12A in the sheath 16, and the load distance of the clip 12, thereby allowing the foremost clip 12A to be surely brought into the usable state where the skirt portions 38 are open. In this manner, it is possible to prevent a situation in which the foremost clip 12A does not completely protrude from the sheath 16 and cannot be used, and to surely bring the foremost clip 12A in the sheath 16 into the usable state.

As described above, in the present invention, the manipulating wire 20 is pushed out toward the forward end by the predetermined length at a time in order to bring the clips 12 into the usable state. In contrast to the case where, e.g., the sheath 16 is pulled toward the proximal end, the present invention has substantially no possibility that friction occurs between the manipulating wire 20 and a member covering the manipulating wire in accordance with the movement of the manipulating wire 20.

The clipping device 10 described above is loaded in a package for a predetermined clip according to a predetermined method.

While the successive clipping device according to the present invention has thus been described in detail, the present invention is not limited to the embodiments described above. It is needless to say that various improvements and modifications may be made in the present invention within the scope not departing from the gist thereof. The successive clipping device of the present invention is usable not only for a flexible endoscope, but also for a rigid endoscope.

## Claims

1. A successive clipping device, comprising:
a plurality of clips each loaded in a forward end portion of a sheath, while being engaged with another of the clips connected respectively in a front-back direction;
a plurality of connection rings corresponding to the plurality of individual clips, fitted in the sheath so as to be capable of advancing and retreating therein, and each covering an engagement portion between the corresponding clips to retain the clips in a connection state;
a manipulating wire inserted in and through the sheath, and having a forward end portion thereof connected to the rearmost clip to pull a clip series including the plurality of clips; and
a manipulating portion connected to a proximal end of the sheath to push out, for the foremost clip that has retreated in the sheath, the manipulating wire with respect to the sheath toward a forward end of the sheath by a first amount of movement necessary for causing the foremost clip to protrude from the forward end of the sheath so as to bring the foremost clip into a usable state, and push out, for the subsequent clip, the manipulating wire with respect to the sheath toward the forward end of the sheath by a second amount of movement smaller than the first amount of movement, and necessary for causing the subsequent clip to protrude from the forward end of the sheath so as to bring the subsequent clip into the usable state.

2. The successive clipping device according to Claim 1, further comprising:
a connecting member attached to the forward end portion of the manipulating wire; and
a dummy clip engaged with the rearmost clip, and connected to the connecting member,
wherein the manipulating portion pushes out, for the dummy clip, the manipulating wire with respect to the sheath toward the forward end of the sheath by a third amount of movement different from the first amount of movement and the second amount of movement, and necessary for causing the dummy clip to protrude from the forward end of the sheath so as to bring the dummy clip into a retrievable state.

3. The successive clipping device according to Claim 1 or 2,
wherein the manipulating portion comprises:
a wire roll obtained by winding a proximal end portion of the manipulating wire;
a case connected to a proximal end portion of the sheath, and accommodating therein the wire roll; and
a wire push-out mechanism for pulling the manipulating wire out of the wire roll to push out the manipulating wire toward the forward end of the sheath.

4. The successive clipping device according to Claim 3,
wherein the wire push-out mechanism comprises:
a scale connected to the manipulating wire, and accommodated in the case;
a dial rotatably attached to the case; and
an interlocking mechanism for moving the scale in a longitudinal direction of the manipulating wire in association with rotation of the dial.

5. The successive clipping device according to Claim 4,
wherein the interlocking mechanism comprises a roller abutted on a side surface of the scale and for moving the scale along the longitudinal direction of the manipulating wire by rotating with the rotation of the dial.

6. The successive clipping device according to Claim 5,
wherein a plurality of recesses arranged at respective intervals corresponding to the first amount of movement and the second amount of movement are formed in the side surface of the scale, and the roller is engaged with each of the plurality of recesses in accordance with movement of the scale.

7. The successive clipping device according to Claim 5,
wherein a plurality of stepped portions arranged at respective intervals corresponding to the first amount of movement and the second amount of movement are formed in the side surface of the scale, and the roller is engaged with each of the plurality of stepped portions in accordance with movement of the scale.

8. The successive clipping device according to any one of Claims 3 to 7, wherein the manipulating portion comprises a wire send-out mechanism for moving the wire roll with respect to the case in an extending direction of the manipulating wire.

9. The successive clipping device according to Claim 8, wherein the wire send-out mechanism comprises:
a tray accommodated in the case, and supporting the wire roll;
a connecting element having one end thereof connected to the tray, and another end thereof extending to an outside of the case; and
a manipulator element connected to the another end of the connecting element.
